# EUROPÄISCHE PATENTSCHRIFT

(11) **EP 1 651 098 B1**
(45) Veröffentlichungstag und Bekanntmachung des Hinweises auf die Patenterteilung: **10.09.2014**
(21) Anmeldenummer: 04741202.8
(22) Anmeldetag: 21.07.2004
(51) Int. Cl.: A61B 1/12, A61B 18/00

(54) **Endoskopisches chirurgisches System**
Endoscopic surgical system
Système endoscopique chirurgical

(30) Priorität: 29.07.2003 DE 10334562
(43) Veröffentlichungstag der Anmeldung: 03.05.2006
(73) Patentinhaber: ERBE Elektromedizin GmbH, 72072 Tübingen (DE)
(72) Erfinder: GEISELHART, Franz, 72770 Reutlingen (DE)
(74) Vertreter: Bohnenberger, Johannes
(86) Internationale Anmeldenummer: PCT/EP2004/008164
(87) Internationale Veröffentlichungsnummer: WO 2005/011483

(56) Entgegenhaltungen:
- EP-A- 1 293 169
- DE-A- 4 332 070
- US-A- 4 402 310
- US-A- 4 509 507
- US-A- 5 785 521
- US-A1- 2001 039 370

## Beschreibung

Die Erfindung betrifft ein endoskopisches chirurgisches System nach dem Oberbegriff des Patentanspruches 1 sowie ein Verfahren zum Betreiben einer Spülvorrichtung für ein chirurgisches Instrument.

Aus der DE 41 39 029 A1 (US 5,720,745) ist ein HF-chirurgisches Gerät bekannt, das zum Koagulieren von Gewebe dient und das zusammen mit einem Endoskop verwendet wird. Hierbei wird Edelgas durch eine Sonde mit einer darin befindlichen Elektrode zur Operationsstelle geleitet und ein HF-Koagulationsstrom der Elektrode und dem Gewebe zugeführt, so dass sich zwischen der Elektrode und dem Gewebebereich ein Plasmastrom bildet, der das Gewebe koaguliert. Zum Säubern der Operationsstelle wird vor, während und nach der Koagulationsbehandlung eine Spülflüssigkeit durch einen Arbeitskanal des Endoskops zugeführt. Ein Spülen der Optik des Endoskops kann ebenfalls erfolgen, um das Sichtfeld freizuhalten.

Aus der WO 01/08577 ist ein chirurgisches Instrument mit einer Elektrode bekannt, die als Polypektomie-Schlinge ausgebildet ist. Argon-Gas wird als Schutzgas an den Operationsort geführt, um die Entstehung von Rauch beim Abtragen eines Polypen mittels HF-Stromes zu verhindern. Eine Spülvorrichtung mit den oben bereits genannten Aufgaben ist ebenfalls vorhanden.

Eine Vielzahl von weiteren chirurgischen Geräten, insbesondere zur Anwendung innerhalb von Körperhöhlen (so z. B. auch Laser-Geräte) sind (s. z.B. US 4 509 507 A1) bekannt, die zu den o. g. Zwecken Inertgas zum Behandlungsbereich leiten und Spüleinrichtungen aufweisen. Alle diese Geräte sind sehr aufwändig gebaut.

Aus der DE 34 15 837 C2 ist eine Flüssigkeitszufuhrvorrichtung für Endoskope bekannt mit einer Luftzufuhrpumpe, über die ein Flüssigkeitsbehälter unter Druck gesetzt werden kann, um dem Endoskop Spülflüssigkeit und - alternativ - Luft zuzuführen. Auch dieses Gerät ist aufwändig aufgebaut.

Aus der DE 43 32 070 A1 ist eine Einrichtung zum Spülen einer Körperhöhle bekannt, bei welcher eine in einem Einwegbeutel enthaltene Spülflüssigkeit in einem Druckbehälter mittels eines von einer Druckquelle erzeugten Luftdrucks dem Spülinstrument zugeleitet wird. Diese Vorrichtung ist ebenfalls aufwändig.

Aus der US 5 785 521 A ist ein chirurgisches Instrument (in diesem Fall ein laserchirurgisches Instrument) nach dem Oberbegriff des Patentanspruches 1 bekannt. Der Spülvorgang ist allerdings bei diesem Gerät nicht exakt steuerbar.

Näheres zum Stand der Technik enthält das US-Patent 4,509,507, das eine Endoskopvorrichtung betrifft. Mittels einer gemeinsamen Pumpe wird entweder Luft oder - aufgrund des von der Pumpe erzeugten Luftdrucks - Wasser dem Endoskop zugeführt. Mittels eines Schalters wird der Luft- bzw. Wasserdruck geändert. Der Luftkanal öffnet über eine Lecköffnung zur Außenwelt. Wird die Lecköffnung vom Operateur mit dem Finger geschlossen, so fließt die Luft durch das Endoskop. Die Lecköffnung befindet sich in einem kolbenförmigen Schaltventil. Durch Betätigung des Schaltventils wird der Luftkanal geschlossen, so dass Wasser dem Endoskop zugeführt wird.

US 2001/0039370 A1 betrifft ein Luft- und Wasserzufuhrsystem für Endoskope. Luft oder Wasser wird mit einer Pumpe zugeführt. Die Wasser- bzw. Wasserflussraten werden jedoch individuell mittels einer Vielzahl an Ventilen gesteuert. US-Patent 4,402,310 enthält eine ähnliche Lehre.

Der Erfindung liegt die Aufgabe zu Grunde, ein System der eingangs genannten Art aufzuzeigen, das vereinfacht aufgebaut und dennoch variabel bedienbar ist bzw. ein entsprechendes Verfahren aufzuzeigen.

Diese Aufgabe wird durch ein endoskopisches chirurgisches System nach Anspruch 1 gelöst.

Die bei einem derartigen System ohnehin vorhandene Gaszufuhreinrichtung wird gemäß einem Grundgedanken der vorliegenden Erfindung dazu benutzt, um den Spüldruck zu erzeugen, so dass gesonderte Pumpeinrichtungen für die Spülflüssigkeit entfallen können. Dies bedeutet nicht nur im Aufbau des Gerätes eine wesentliche Vereinfachung, vielmehr werden auch der Betrieb vereinfacht und die Ausfallsicherheit erhöht, da gesonderte Pumpeinrichtungen vermieden werden können. Weiterhin weist die Gaszufuhreinrichtung eine Regeleinrichtung zum Regeln eines Druckes und/oder eines Volumenstroms des Inertgases auf, so dass durch diese Regeleinrichtung der "Vordruck" der Spülflüssigkeit in einfacher Weise derart steuerbar ist, dass der Druck- und/oder Volumenstrom zwischen mindestens einem ersten Wert zum Zuführen des Inertgases zum Gewebe-Behandlungsbereich und einem zweiten Wert zum Erzeugen des Spüldruckes umschaltbar ist. Dadurch kann die Regeleinrichtung in zweifacher Weise verwendet werden, nämlich zum einen, um den Druck des Gases zu regeln, welches dem Behandlungsbereich zugeführt wird und zum anderen die Spülflüssigkeit unter einen davon verschiedenen Druck zu setzen, wie er zum Spülen benötigt wird.

Vorzugsweise ist eine zweite Regeleinrichtung zum Regeln eines Druckes und/oder Volumenstroms der Spülflüssigkeit vorgesehen. Dadurch kann der Spülvorgang - ausgehend von einem maximalen Vordruck - so eingestellt werden, dass das vom Operateur gewünschte Ziel exakt erreicht wird.

Vorzugsweise ist eine Filtereinrichtung zum keimfreien Filtrieren des Inertgases vorgesehen. Dies erhöht die Sicherheit des Patienten.

Gemäß der Erfindung ist ein Druckbehälter zum Einfüllen der Spülflüssigkeit und zum Einleiten des unter Druck stehenden Inertgases derart vorgesehen, dass der Druck des Inertgases auf die Spülflüssigkeit wirkt. Diese Einwirkung des Druckes auf die Spülflüssigkeit kann in dem Druckbehälter direkt geschehen, indem der Druckbehälter mit einem Steigrohr (für die Spülflüssigkeit) ausgestattet wird. Vorzugsweise wird jedoch ein Innenbehälter zur Aufnahme der Spülflüssigkeit vorgesehen, der eine elastische Wand derart aufweist, dass die Spülflüssigkeit durch die elastische Wand von dem Inertgas getrennt ist. Der Druckbehälter kann somit am Ort (z. B. in einem fahrbaren Gestell für das chirurgische Instrument) verbleiben, wenn der Spülflüssigkeitsbehälter ausgetauscht wird. Eine Kontamination der Spülflüssigkeit durch das Inertgas oder durch den Druckbehälter ist somit ausgeschlossen. Besonders bevorzugt wird hierbei der Innenbehälter als handelsüblicher Infusionsbeutel ausgebildet. Derartige Infusionsbeutel, die mit einer als Spülflüssigkeit geeigneten, sterilen Lösung gefüllt sind, sind in jedem Krankenhaus vorhanden.

Bei einer bevorzugten Ausführungsform der Erfindung ist eine Mischeinrichtung vorgesehen, zum Mischen des Inertgases mit der Spülflüssigkeit. Auf diese Weise kann die Menge der Spülflüssigkeit vermindert werden, ohne dabei die Reinigungswirkung wesentlich zu verschlechtern. Dies ist beispielsweise dadurch möglich, dass die Mischeinrichtung eine Schaumerzeugungseinrichtung umfasst, so dass aus einer Düse der Spüleinrichtung Schaum ausgespritzt wird, dessen Bläschen nach dem Austreten aus der Düse platzen und dadurch einen in feine Tröpfchen aufgeteilten Spülstrom erzeugen.

Alternativ oder auch zusätzlich kann die Mischeinrichtung eine Umschalteinrichtung umfassen zum Umschalten zwischen Inertgas und Spülflüssigkeit, die derart ausgebildet ist, dass abwechselnd Inertgas und Spülflüssigkeit dem chirurgischen Instrument zugeführt werden. Dadurch kommt es zu einer weiteren Verringerung der Spülflüssigkeitsmenge. Die Umschalteinrichtung ist vorzugsweise hierbei derart ausgebildet, dass im Wesentlichen gleich große Spülmittelvolumina durch den Druck des Inertgases beschleunigt und nacheinander auf den Zielbereich "geschossen" werden. Eine derartige Spüleinrichtung ist beispielsweise aus der DE 195 45 528 (US 6,428,507) an sich bekannt.

Vorzugsweise ist das chirurgische Instrument ein HF-chirurgisches Instrument, da bei derartigen Geräten, insbesondere bei Anwendung in Körperhöhlen eine Druckgasquelle, nämlich eine Druckflasche mit Argon-Gas vorhanden ist. Ebenso ist bevorzugt, das chirurgische Instrument als laserchirurgisches Instrument auszubilden, da auch dort mit großem Vorteil ein Inertgas Verwendung findet.

Bei einem Verfahren zum Betreiben einer Spülvorrichtung für ein chirurgisches System, wird ein unter Druck stehendes Gas einem Spülflüssigkeitsbehälter derart zugeführt, dass die Spülflüssigkeit unter einen Spüldruck gesetzt wird. Man kann also auch Druckluft als Druckgas verwenden, wie sie in Operationssälen üblicherweise zur Verfügung steht. Dies vereinfacht die Druckerzeugung insbesondere dann, wenn - wie oben beschrieben - die Spülflüssigkeit in einem elastischen Behälter (insbesondere einem Infusionsbeutel) vorliegt und in einen Druckbehälter eingesetzt wird, der dann unter den Gasdruck gesetzt wird.

Bevorzugte Ausführungsformen der Erfindung ergeben sich aus den Unteransprüchen und der nachfolgenden Beschreibung der Erfindung, die an Hand von Abbildungen näher erläutert wird. Hierbei zeigen
- Fig. 1: einen Prinzipaufbau einer ersten Ausführungsform der Erfindung,
- Fig. 2: eine Darstellung ähnlich der nach Fig. 1 zur Erläuterung einer weiteren Ausführungsform der Erfindung und
- Fig. 3: eine Darstellung ähnlich der nach Fig. 1 zur Erläuterung einer dritten Ausführungsform der Erfindung.

In der nachfolgenden Beschreibung werden gleiche und gleich wirkende Teile mit den selben Bezugsziffern versehen.

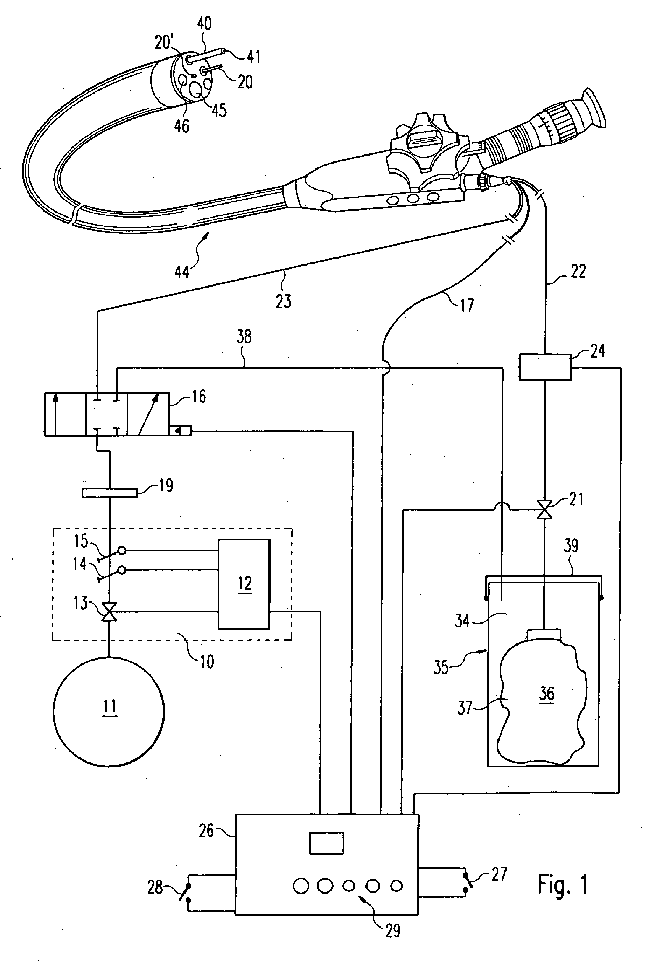

In Fig. 1 ist ein chirurgisches Gerät in einer Prinzipdarstellung gezeigt, das mit einem Endoskop 44 verwendet wird. Es handelt sich hierbei um eine sog. APC-Sonde (Argon-Plasma-Chirurgie-Sonde), wie sie beispielsweise aus der DE 41 39 029 A1 (US 5,720,745) bekannt ist.

Wie in Fig. 1 angedeutet, weist das Endoskop 44 Arbeitskanäle 46 auf, wobei aus einem Arbeitskanal das eigentliche chirurgische Instrument 40, nämlich eine APC-Sonde mit ihrem als Funktionsabschnitt 41 ausgebildeten offenen Ende und aus einem anderen Arbeitskanal eine Spüleinrichtung 20 hervorragt. Weiterhin ist für eine Optik 45 des Endoskops 44 eine Spüleinrichtung 20' vorgesehen.

Zur Zufuhr von Edelgas, insbesondere Argon oder Helium aus einer Gasflasche 11 über eine Gaszufuhreinrichtung 10 und eine Gasleitung 23 zur APC-Sonde 40 ist in der Gaszufuhreinrichtung 10 ein Proportionalventil 13 vorgesehen, welches von einer ersten Regeleinrichtung 12 in Übereinstimmung mit Signalen aus einem Druckfühler 14 und einem Volumenstromfühler 15 geregelt wird. Der durch ein Filter 19 und ein Dreiwegeventil 16 (in dessen linker Stellung) gelangende Gasstrom wird über die erste Regeleinrichtung 12 hinsichtlich seines Druckes und seines Volumens pro Zeiteinheit derart geregelt, dass ein gleichmäßiger und den Anwendungszwecken entsprechender Argonstrom aus der APC-Sonde 40 ausströmt.

Zur Einstellung der Werte (Druck, Volumenstrom, An/Aus) des Gasstromes sowie eines HF-Koagulationsstromes, der über eine HF-Stromzufuhr 17 der APC-Sonde 40 zugeleitet wird, ist ein Chirurgiegerät 26 vorgesehen, das Einstellorgane 29 und einen (Fuß-) Schalter 28 aufweist. Bei Betätigung des Fußschalters 8 wird zunächst das Ventil 16 derart betätigt, dass der Gasstrom durch die Leitung 23 in die APC-Sonde 40 strömen kann, bevor der Koagulationsstrom eingeschaltet wird. Dies ist an sich bekannt.

Weiterhin ist eine Druckleitung 38 vorgesehen, die (in der rechten Stellung des Ventils 16) das unter Druck stehende Inertgas in einen Druckbehälter 35 führt, der durch einen Deckel 39 gasdicht verschlossen ist. Im Druckbehälter 35 befindet sich ein Innenbehälter 36, insbesondere ein mit Ringer-Lösung gefüllter Infusionsbeutel, der eine elastische Wand 37 aufweist. Eine Spülleitung 22 ist an den Innenbehälter 36 derart angeschlossen, dass bei Einleiten eines Druckes in den Druckbehälter 35 die elastische Wand 37 des Innenbehälters 36 eingedrückt und somit in dem Innenbehälter befindliche Flüssigkeit in die Spülleitung 22 gedrückt wird.

Weiterhin sind in die Spülleitung 22 ein Spülventil 21 (An/Aus) und eine zweite Regeleinrichtung 24 eingeschaltet, die beide über entsprechende Steuerleitungen vom Chirurgiegerät 26 steuerbar sind. Das Spülventil 21 kann durch einen (z. B. mit dem Fuß zu betätigenden) Spülschalter 27 an- und ausgeschaltet werden, während die Strömungsparameter, insbesondere der Druck und der Volumenstrom (Volumen/Zeit) der Spüllösung über die Einstellorgane 29 des Chirurgiegerätes 26 und die zweite Regeleinrichtung 24 einstellbar sind.

Das Ventil 26 wird vorzugsweise derart betätigt, dass das Inertgas entweder zum chirurgischen Instrument 40 oder aber zum Druckbehälter 35 geleitet wird. Alternativ ist es natürlich möglich, die Leitung 38 direkt nach dem Filter 49 oder gar an die Gasflasche 11 anzuschließen, so dass entweder der durch die erste Regeleinrichtung 12 bestimmte Druck oder aber der Gasflaschendruck (ggf. über ein Druckmindeterventil) einem Innenraum des Druckbehälters 35 zugeleitet wird und die Steuerung des Spülflüssigkeitsstroms ausschließlich durch das Spülventil 21 bzw. die zweite Regeleinrichtung 24 erfolgt.

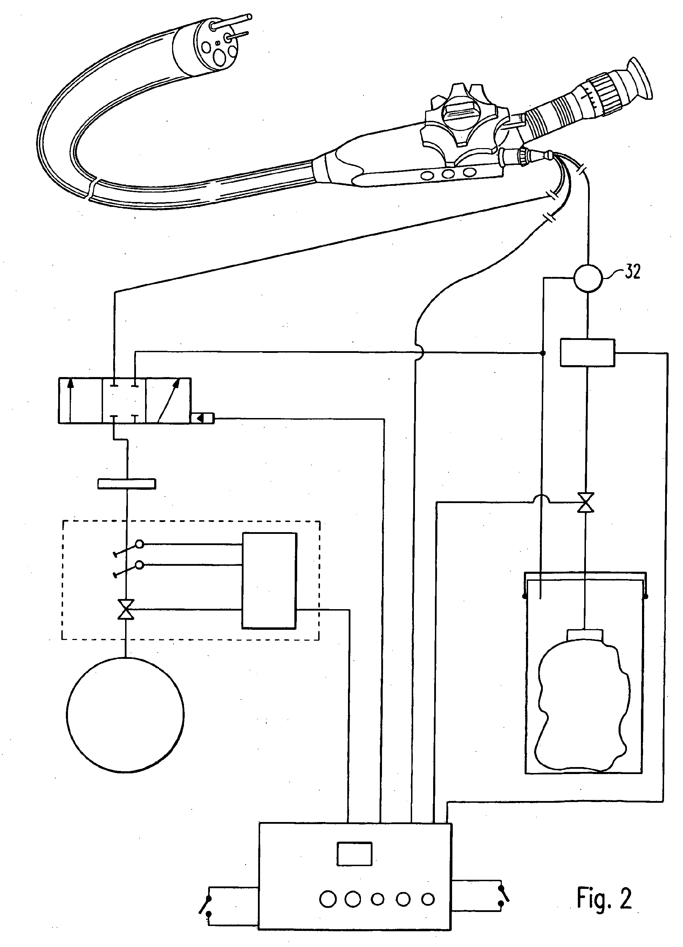

Bei der in Fig. 2 gezeigten Ausführungsform ist zusätzlich zu den in Fig. 1 gezeigten Teilen eine Schaumerzeugungseinrichtung 32 vorgesehen, welche die Spülflüssigkeit mit Inertgas mischt, um so eine Verminderung der Spülflüssigkeitsmenge bei nur unwesentlich veränderten Spüleigenschaften zu erreichen.

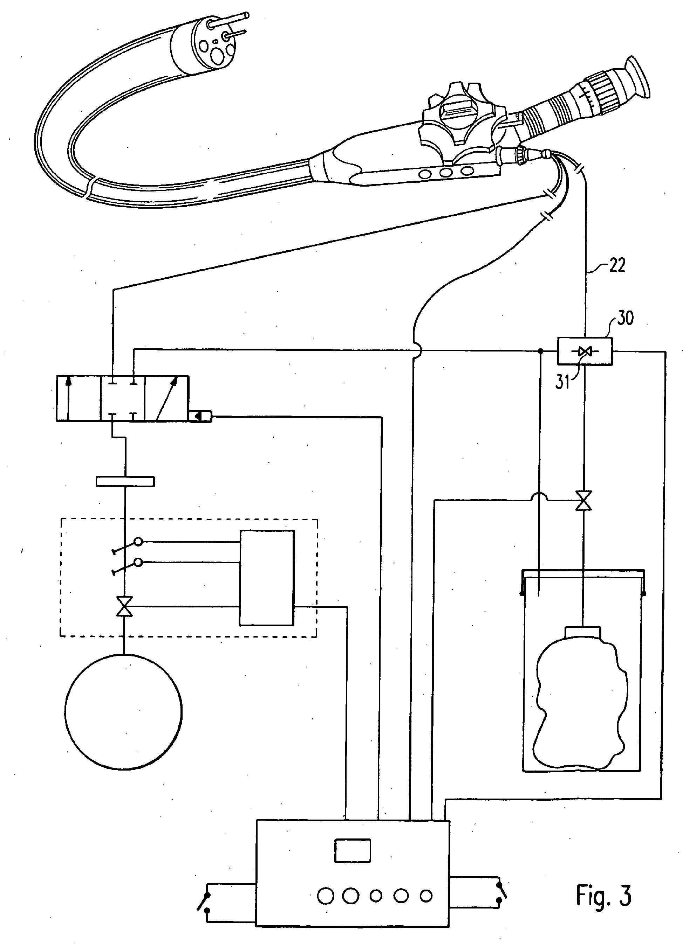

Bei der in Fig. 3 gezeigten Ausführungsform der Erfindung ist an Stelle der Schaumerzeugungseinrichtung 32 eine Mischeinrichtung 30 vorgesehen, welche eine Umschalteinrichtung 31 aufweist, um zwischen Inertgas und Spülflüssigkeit derart umzuschalten, dass kleine "Spülflüssigkeitspropfen" durch die Spülleitung 22 mit dahinter liegenden Druckgas-Volumina geführt und damit beschleunigt werden, bis sie aus der Spüleinrichtung 20 bzw. deren Ende ausgestoßen werden. Dies kann selbstverständlich auch mit der Ausführungsform nach Fig. 2 derart kombiniert werden, dass "Schaumpropfen" durch nachfolgende Gaspolster beschleunigt und ausgestoßen werden. Die in Fig. 1 gezeigte Regelung des Spüldruckes durch die zweite Regeleinrichtung 24 kann hier ebenfalls erfolgen.

### Bezugszeichenliste

- 10: Gaszufuhreinrichung
- 11: Gasflasche
- 12: 1. Regeleinrichtung
- 13: Ventil
- 14: Druckfühler
- 15: Volumenstromfühler
- 16: Dreiwegeventil
- 17: HF-Stromzufuhr
- 19: Filter
- 20, 20': Spüleinrichtung
- 21: Spülventil
- 22: Spülleitung
- 23: Gasleitung
- 24: 2. Regelventil
- 26: Chirurgiegerät
- 27: Spülschalter
- 28: Fußschalter
- 29: Einstellorgan
- 30: Mischeinrichtung
- 31: Umschalteinrichtung
- 32: Schaumerzeugungseinrichtung
- 34: Innenraum
- 35: Druckbehälter
- 36: Innenbehälter
- 37: Wand
- 38: Druckleitung
- 39: Deckel
- 40: Chirurgisches Instrument
- 41: Funktionsabschnitt
- 44: Endoskop
- 45: Optik
- 46: Arbeitskanal

## Patentansprüche

1. Endoskopisches chirurgisches System, umfassend
ein Endoskop (44) mit einer Spüleinrichtung (20) zum Spülen eines Zielbereiches; ein chirurgisches Instrument (40); eine erste Regeleinrichtung (12);
eine mit dem Endoskop (44) verbundene Gasleitung (23);
eine Druckleitung (38), die in einen Druckbehälter (35) zum Einfüllen von Spülflüssigkeit und zum Einleiten eines unter Druck stehenden Inertgases mündet;
eine Gaszufuhreinrichtung (10) zum Zuführen des Inertgases; und
ein zwischen der Gaszuführeinrichtung (10), der Gasleitung (23) und der Druckleitung (38) geschaltetes Dreiwegeventil (16), wobei die Spüleinrichtung (20) den Zielbereich mit einem Spülstrom der Spülflüssigkeit spült, die unter einem Spüldruck steht, welcher Spüldruck über die Druckleitung (38) durch das Inertgas von der Gaszufuhreinrichtung (10) erzeugt wird, wobei
die erste Regeleinrichtung (12) derart steuerbar ist, dass der Druck und/oder der Volumenstrom zwischen mindestens einem ersten Wert zum Zuführen des Inertgases zum Gewebe-Behandlungsbereich und einem zweiten Wert zum Erzeugen des Spüldruckes umschaltbar ist, und
das Dreiwegeventil in einer ersten Stellung die Gaszufuhreinrichtung lediglich mit der Gasleitung verbindet, in einer zweiten Stellung die Gaszufuhreinrichtung, die Gasleitung und die Druckleitung voneinander trennt und in einer dritten Stellung die Gaszufuhreinrichtung lediglich mit der Druckleitung verbindet,
**dadurch gekennzeichnet, dass**
der Druckbehälter (35) einen Innenbehälter (36) zur Aufnahme der Spülflüssigkeit umfasst, der eine elastische Wand (37) derart aufweist, dass die Spülflüssigkeit durch die elastische Wand (37) von dem Inertgas getrennt ist, wobei das Inertgas auf die Spülflüssigkeit wirkt.

2. Endoskopisches chirurgisches System nach einem der vorhergehenden Ansprüche,
**gekennzeichnet durch**
eine zweite Regeleinrichtung (24) zum Regeln eines Druckes und/oder Volumenstroms der Spülflüssigkeit.

3. Endoskopisches Chirurgisches System nach einem der vorhergehenden Ansprüche,
**gekennzeichnet durch**
ein Spülventil (21) zum An- und Ausschalten des Spülstromes bei vorliegendem Spüldruck.

4. Endoskopisches Chirurgisches System nach einem der vorhergehenden Ansprüche,
**gekennzeichnet durch**
eine Filtereinrichtung (19) zum keimfreien Filtrieren des Inertgases.

5. Endoskopisches Chirurgisches System nach Anspruch 1,
**dadurch gekennzeichnet, dass**
**der** Innenbehälter (36) als handelsüblicher Infusionslösungsbeutel ausgebildet ist.

6. Endoskopisches Chirurgisches System nach einem der vorhergehenden Ansprüche,
**gekennzeichnet durch**
eine Mischeinrichtung (30, 32) zum Mischen des Inertgases mit der Spülflüssigkeit.

7. Endoskopisches Chirurgisches System nach Anspruch 6,
**dadurch gekennzeichnet, dass**
die Mischeinrichtung (32) eine Schaumerzeugungseinrichtung umfasst.

8. Endoskopisches Chirurgisches System nach einem der Ansprüche 6 oder 7,
**dadurch gekennzeichnet, dass**
die Mischeinrichtung (30) eine Umschalteinrichtung (31) zum Umschalten zwischen Inertgas und Spülflüssigkeit umfasst, die derart ausgebildet ist, dass abwechselnd Inertgas und Spülflüssigkeit dem chirurgischen Instrument (40) zugeführt werden.

9. Endoskopisches Chirurgisches System nach Anspruch 8,
**dadurch gekennzeichnet, dass**
die Umschalteinrichtung (31) derart ausgebildet ist, dass im Wesentlichen gleich große Spülflüssigkeitsvolumina durch den Druck des Inertgases beschleunigt und nacheinander auf den Zielbereich "geschossen" werden.

10. Endoskopisches Chirurgisches System nach einem der vorhergehenden Ansprüche,
**dadurch gekennzeichnet, dass**
das chirurgische Instrument (40) ein HF-chirurgisches Gerät ist.

11. Endoskopisches Chirurgisches System nach einem der Ansprüche 1 - 9,
**dadurch gekennzeichnet, dass**
das Gerät ein laserchirurgisches Gerät ist.

## Claims

1. Endoscopic surgical system, comprising
an endoscope (44) with a rinsing device (20) for rinsing a target region;
a surgical instrument (40);
a first regulator (12);
a gas line (23) connected to the endoscope (44);
a pressure line (38), which opens into a pressure container (35) for introducing rinsing liquid and for introducing a pressurized inert gas;
a gas delivery device (10) for delivering the inert gas; and
a three-way valve (16) connected between the gas delivery device (10), the gas line (23) and the pressure line (38), wherein
the rinsing device (20) rinses the target region with a rinsing stream of the rinsing liquid, which is at a rinsing pressure, said rinsing pressure being generated via the pressure line (38) by the inert gas from the gas delivery device (10), wherein
the first regulator (12) can be controlled in such a way that the pressure and/or the volumetric flow can be switched between at least a first value, for delivering the inert gas to the tissue treatment region, and a second value, for generating the rinsing pressure, and
the three-way valve, in a first position, connects the gas delivery device only to the gas line, in a second position separates the gas delivery device, the gas line and the pressure line from one another, and, in a third position, connects the gas delivery device only to the pressure line, **characterized in that**
the pressure container (35) comprises an inner receptacle (36) for receiving the rinsing liquid, which inner receptacle (36) has an elastic wall (37), such that the rinsing liquid is separated from the inert gas by the elastic wall (37), wherein the inert gas acts on the rinsing liquid.

2. Endoscopic surgical system according to the preceding claim, **characterized by** a second regulator (24) for regulating a pressure and/or volumetric flow of the rinsing liquid.

3. Endoscopic surgical system according to one of the preceding claims, **characterized by** a rinsing valve (21) for switching the rinsing stream on and off at the existing rinsing pressure.

4. Endoscopic surgical system according to one of the preceding claims, **characterized by** a filter device (19) for filtering the inert gas so that the latter is germ-free.

5. Endoscopic surgical system according to Claim 1, **characterized in that** the inner receptacle (36) is designed as a commercially available infusion solution bag.

6. Endoscopic surgical system according to one of the preceding claims, **characterized by** a mixer (30, 32) for mixing the inert gas with the rinsing liquid.

7. Endoscopic surgical system according to Claim 6, **characterized in that** the mixer (32) comprises a foam generator.

8. Endoscopic surgical system according to either of Claims 6 and 7, **characterized in that** the mixer (30) comprises a switch (31) for switching between inert gas and rinsing liquid, which is designed to deliver inert gas and rinsing liquid alternately to the surgical instrument (40).

9. Endoscopic surgical system according to Claim 8, **characterized in that** the switch (31) is designed in such a way that substantially equal volumes of rinsing liquid are accelerated by the pressure of the inert gas and are "shot" one after another onto the target region.

10. Endoscopic surgical system according to one of the preceding claims, **characterized in that** the surgical instrument (40) is a high-frequency surgical appliance.

11. Endoscopic surgical system according to one of Claims 1 to 9, **characterized in that** the appliance is a laser-surgery appliance.

## Revendications

1. Système endoscopique chirurgical, comprenant:
- un endoscope (44) avec un dispositif de balayage (20) pour le balayage d'une zone de cible;
- un instrument chirurgical (40);
- un premier dispositif de régulation (12);
- une conduite de gaz (23) raccordée à l'endoscope (44);
- une conduite sous pression (38), qui débouche dans un réservoir sous pression (35) pour charger du liquide de balayage et pour introduire un gaz inerte se trouvant sous pression;
- un dispositif d'amenée de gaz (10) pour amener le gaz inerte; et
- une vanne à trois voies (16), connectée entre le dispositif d'amenée de gaz (10), la conduite de gaz (23) et la conduite sous pression (38), dans lequel
- le dispositif de balayage (20) balaie la zone de cible avec un courant de balayage du liquide de balayage, qui se trouve sous une pression de balayage, pression de balayage qui est produite via la conduite sous pression (38) par le gaz inerte venant du dispositif d'amenée de gaz (10), dans lequel
- le premier dispositif de régulation (21) peut être commandé de telle manière que la pression et/ou le courant volumique puisse être commuté(e) entre au moins une première valeur pour amener le gaz inerte jusqu'à la zone de traitement de tissu et une deuxième valeur pour produire la pression de balayage, et
- la vanne à trois voies relie dans une première position le dispositif d'amenée de gaz uniquement à la conduite de gaz, sépare dans une deuxième position le dispositif d'amenée de gaz, la conduite de gaz et la conduite sous pression l'une de l'autre et relie dans une troisième position le dispositif d'amenée de gaz uniquement à la conduite sous pression,
**caractérisé en ce que** le réservoir sous pression (35) comprend un réservoir intérieur (36) destiné à contenir le liquide de balayage, qui présente une paroi élastique (37) de telle manière que le liquide de balayage soit séparé du gaz inerte par la paroi élastique (37), le gaz inerte agissant sur le liquide de balayage.

2. Système endoscopique chirurgical selon la revendication précédente, **caractérisé par** un deuxième dispositif de régulation (24) pour réguler une pression et/ou un courant volumique du liquide de balayage.

3. Système endoscopique chirurgical selon l'une quelconque des revendications précédentes, **caractérisé par** une soupape de balayage (21) pour connecter et déconnecter le courant de balayage lorsque la pression de balayage est présente.

4. Système endoscopique chirurgical selon l'une quelconque des revendications précédentes, **caractérisé par** un dispositif de filtrage (19) pour le filtrage sans germes du gaz inerte.

5. Système endoscopique chirurgical selon la revendication 1, **caractérisé en ce que** le réservoir intérieur (36) se présente sous la forme d'une poche pour solution d'infusion disponible dans le commerce.

6. Système endoscopique chirurgical selon l'une quelconque des revendications précédentes, **caractérisé par** un dispositif de mélange (30, 32) pour mélanger le gaz inerte avec le liquide de balayage.

7. Système endoscopique chirurgical selon la revendication 6, **caractérisé en ce que** le dispositif de mélange (32) comprend un dispositif de production de mousse.

8. Système endoscopique chirurgical selon l'une des revendications 6 ou 7, **caractérisé en ce que** le dispositif de mélange (30) comprend un dispositif de commutation (31) pour la commutation entre gaz inerte et liquide de balayage, qui est configuré de telle manière que du gaz inerte et du liquide de balayage soient envoyés en alternance à l'instrument chirurgical (40).

9. Système endoscopique chirurgical selon la revendication 8, **caractérisé en ce que** le dispositif de commutation (31) est configuré de telle manière que des volumes de liquide de balayage essentiellement égaux soient accélérés par la pression du gaz inerte et projetés l'un après l'autre sur la zone de cible.

10. Système endoscopique chirurgical selon l'une quelconque des revendications précédentes, **caractérisé en ce que** l'instrument chirurgical (40) est un appareil chirurgical HF.

11. Système endoscopique chirurgical selon l'une quelconque des revendications 1 à 9, **caractérisé en ce que** l'appareil est un appareil de chirurgie au laser.
